Europäisches Patentamt

European Patent Office

Office européen des brevets

(11) Publication number: **0 053 031**
**B1**

⑩

(12) **EUROPEAN PATENT SPECIFICATION**

(45) Date of publication of patent specification: **12.09.84**

(21) Application number: **81305529.0**

(22) Date of filing: **24.11.81**

(51) Int. Cl.³: **C 07 C 79/10, C 07 C 79/12, C 07 C 76/02**

(54) A process for the vapor phase nitration of aromatic compounds.

(30) Priority: **26.11.80 US 210423**

(43) Date of publication of application:
**02.06.82 Bulletin 82/22**

(45) Publication of the grant of the patent:
**12.09.84 Bulletin 84/37**

(84) Designated Contracting States:
**DE FR GB IT**

(56) References cited:
**EP-A-0 001 922**
**GB-A-2 000 141**
**US-A-3 966 830**

(73) Proprietor: **MONSANTO COMPANY**
**Patent Department 800 North Lindbergh Boulevard**
**St. Louis, Missouri 63166 (US)**

(72) Inventor: **Schumacher, Ignatius**
**336 West Clayton Road**
**Ballwin Missouri 63011 (US)**
Inventor: **Wang, Kang-bo**
**13386 Primwood Drive**
**Creve Coeur Missouri 63141 (US)**

(74) Representative: **Pearson, John Lionel et al**
**Monsanto Europe S.A. Avenue de Tervuren 270-272 Letter Box No 1**
**B-1150 Brussels (BE)**

Courier Press, Leamington Spa, England.

# O 053 031

**Description**

Field of the invention

This invention relates to a process for the nitration of aromatic compounds. More particularly, this invention relates to a process for the vapor phase nitration of aromatic compounds susceptible of existing in the vapor phase at temperatures less than about 190°C. in the presence of a molecular sieve catalyst.

Nitroaromatic compounds find use as solvents, explosives, dyes, perfumes, and analytical reagents, and are important as intermediates in organic synthesis. As an example, nitroaromatic compounds are convertible by reduction into primary amines, which, in turn, are valuable intermediates in the synthesis of dyes, pharmaceuticals, photographic developers, antioxidants, and gum inhibitors.

Description of the prior art

Vapor phase nitration of aromatic compound is known in the art. The vapor phase nitration of benzene and toluene at temperatures ranging from about 275°C. to about 310°C. is described in McKee and Wilhelm, *Industrial and Engineering Chemistry*, *28* (6), 662—667 (1936) and U.S. Patent 2,109,873. McKee and Wilhelm catalyzed their reaction with silica gel, with best results being reported by the use of 14 mesh material.

Bauxite and alumina were reported to be ineffective as catalysts in the vapor phase nitration of benzene.

More recently, U.S. Patent 4,107,220 described the vapor phase nitration of chlorobenzene in the presence of molecular sieve catalysts having a pore size varying from about 5 Å to about 10 Å as a means for controlling the para-to-ortho isomer distribution of nitrochlorobenzene. A suitable temperature range was reported to be from about 190°C. to about 290°C.

Although these prior art processes generally provide the desired product, they nevertheless are limited in their applications. Principal among these limitations, which result from the severe operating conditions, are low (aromatic compound) conversions and contamination of the nitroaromatic compound product by undesirable by-products.

GB—A—2,000,141 describes a method for the nitration of aromatic compounds in which the aromatic compound is reacted with nitric acid in the gaseous phase in the presence of an acid aluminosilicate type catalyst at temperatures ranging from 150°C to 250°C, with 170°C to 200°C being preferred. Toluene and benzene are disclosed as suitable aromatic compounds.

US—A—3,966,830 relates to a process for the nitration of halogenated benzene derivatives in the presence of a solid inorganic oxide selected from the group consisting of silica, alumina, and silica-alumina, including those on which have been deposited oxyacids of sulfur and the salts thereof or the oxyacids of phosphorus, that is, sulfuric acid and its salts or orthophosphoric acid.

The present invention provides a process for the vapor phase nitration of aromatic compounds susceptible of existing in the vapor phase at temperatures less than 190°C, which process comprises contacting the aromatic compound with a gaseous oxide of nitrogen higher than nitric oxide as nitrating agent selected from nitrogen dioxide, dinitrogen trioxide, and dinitrogen tetroxide in the vapor phase in the presence of water and a molecular sieve catalyst, characterised in that the process is carried out at a temperature between 80°C and 190°C and the molecular sieve catalyst is conditioned by pretreatment with nitrating agent.

The process is characterized by operation at relatively low temperature, high aromatic compound conversion and high nitro-aromatic compound selectivity.

Non-limiting representative examples of aromatic compounds suitable for use in the present process which are susceptible of existing in the vapor phase at temperatures less than 190°C. (at atmospheric pressures) include aromatic hydrocarbons, such as benzene, toluene, xylenes, ethylbenzene and cumene; aromatic ethers such as anisole and phenetole; and haloaromatic compounds such as chlorobenzene, bromobenzene, iodobenzene. It has been found, however, that the process of this invention is particularly efficacious with chlorobenzene (also known as monochlorobenzene or simply MCB) and benzene.

The nitrating agents which are employed in the process of this invention are the gaseous oxides of nitrogen higher than nitric oxide (NO) such as nitrogen dioxide ($NO_2$), dinitrogen trioxide ($N_2O_3$), and dinitrogen tetroxide ($N_2O_4$). Of these nitrating agents, nitrogen dioxide is preferred. Thus, for convenience and clarity the process will be described, with reference to the preferred nitrogen dioxide as the nitrating agent.

The molecular sieve catalysts employed in accordance with this invention are aluminosilicate compounds having a well-defined crystalline structure; they may or may not be hydrated. The fundamental structural units are silicon and aluminum atoms tetrahedrally coordinated with four oxygen atoms. The silicate and aluminate units are generally joined to form 4- and 6-membered rings of oxygen atoms forming a simple and consistent arrangement of polyhedra. Each polyhedron is a three-dimensional array of tetrahedra in a definite geometric form.

The pore size of the molecular sieve catalyst suitable for use in the present process is not narrowly critical. However, the pore size preferably should be at least 5 Å. The use of a substantially smaller pore

2

size molecular sieve has been found to have substantially reduced effectiveness for producing the nitro-aromatic compound under conditions employed herein to produce the desired product. The maximum pore size of the molecular sieve is not critical and is limited only in terms of practicality and the availability of such molecular sieves.

Illustrative examples of the structure and synthesis of conventional molecular sieve catalysts suitable for use in this invention can be had by reference to U.S. Patent 2,882,243; 2,882,244; 3,130,007; and 3,216,789. For further information thereon and a general review of zeolite molecular sieve catalysts, see Breck, *Zeolite Molecular Sieves*, John Wiley & Sons, New York, NY 1974.

Representative examples of suitable naturally occurring molecular sieve catalysts are analcime, bika-taite, brewsterite, chabazite, clinoptilobite, bachiardite, edingtonite, epistilbite, erionite, faujasite, ferrierite, gismondine, gmelinite, gonnardite, harmontome, heulandite, kieselguhr, laumontite, levynite, losod, mesolite, mordenite, natrolite, omega, paulingite, phillipsite, scolecite, sodalite hydrate, stilbite, thomsonite, yugawaralite, and suitable synthetic zeolites are those zeolite compounds known as "A", "N—A", "L$^h$", "P", "T", "X", "Y", "ZK-4", and "ZK-5".

Of these molecular sieve catalysts, mordenite is preferred. Some commercially available mordenite molecular sieve catalysts are sold under the trademarks Zeolon 900 H (8—9 Å pore size; $SiO_2/Al_2O_3$ molar ratio—10/1) from Norton Company of Akron, Ohio, U.S.A.; and Zeolon 200 H (8—9 Å pore size; $SiO_2/Al_2O_3$ molar ratio—10/1) from Norton Company.

It will be appreciated that the invention is not limited to the aforesaid specific molecular sieves and that other suitable molecular sieves having larger and smaller pore size dimensions as well as larger and smaller $SiO_2/Al_2O_3$ molar ratios can be readily selected by persons skilled in the art in light of the aforesaid disclosures and the specific illustrative examples.

In the process of the invention, the molecular sieve catalyst is conditioned by pretreatment with nitrogen dioxide at operating conditions to the saturation point (in the absence of the aromatic compound). Pretreatment times in general range from 1 minute to 1 hour or more. The actual pretreatment time, however, will depend upon the amount or quantity and pore structure of the molecular sieve catalyst, the flow rate of the nitrogen dioxide and the operating conditions. Usually, pretreatment for 5 minutes to 30 minutes is sufficient.

The conditioning pretreatment permits almost immediate production of the nitroaromatic compound upon introduction of the aromatic compound to the reactor. In the absence of the pretreatment, measurable nitroaromatic compound of production is delayed until the molecular sieve catalyst becomes saturated with nitrogen dioxide.

The vapor phase nitration process of this invention is conducted at temperatures between 80°C. and 190°C., with temperatures between 150°C and 175°C. being preferred in that at such preferred temperatures the rate of reaction is reasonably rapid and little, if any, by-product formation occurs. It will be appreciated, however, that the particular temperatures employed for a given aromatic compound will depend to some extent upon the boiling point or vaporization temperature of the particular aromatic compound. For example, when chlorobenzene, which has a boiling point of 132°C., is the aromatic compound of choice, the vapor phase nitration is conveniently carried out within the aforesaid preferred temperature range, with 175°C. being particularly preferred. When benzene (b.p., 80°C.) is the aromatic compound of choice, the vapor phase nitration may be conducted at temperatures which encompass the entire operative temperature range, that is, from 80°C. to 190°C. Again, however, temperatures between 150°C. and 175°C. are preferred.

The advantages accruing from conducting the vapor phase nitration of this invention at the relatively low elevated temperatures between 80°C. and 190°C. include:

(a) greater selectivity to the desired nitroaromatic compound;
(b) little, if any, by-product formation (to contaminate the desired product);
(c) high material balance between reactants and products;
(d) lower energy requirements; and
(e) minimal thermal decomposition of the nitrogen dioxide.

The latter advantage [(e)] is particularly significant in that it, to a large extent, influences the remaining advantages. It, of course, is well-known in the art that at elevated temperatures nitrogen dioxide undergoes thermal decomposition into the inert (for purposes of this invention) nitric oxide and molecular oxygen. The decomposition begins at about 150°C. and is complete at about 620°C. The decomposition at various tempratures is as follows:

| Temperature, °C. | 130 | 150 | 184 | 279 | 494 | 620 |
|---|---|---|---|---|---|---|
| Decomposition, % | 0 | 3 | 5 | 13 | 56.5 | 100 |

Thus, at temperatures between 80°C. and 190°C., the maximum loss of active nitrogen dioxide by thermal decomposition into inert nitric oxide is only about 5%, while at higher temperatures up to about 300°C., the loss by thermal decomposition rapidly increases to 20—30% or more. Clearly, the magnitude of the loss of nitrogen dioxide at temperatures higher than the operating temperatures of this invention is wasteful and impractical. Moreover, if recirculation of the effluent stream from such high temperature processes is desired, it is necessary to employ an additional step to reoxidize the inert nitric oxide to the active nitrogen dioxide by treatment thereof with oxygen or an oxygen-containing gas such as air, with the attendant added cost and complexity. The additional cost and complexity of this added step, however, is substantially reduced or eliminated altogether by the relatively low temperature conditions employed in the process of this invention.

The vapor phase nitration process of this invention is carried out in the presence of water, which is believed necessary to create and renew reaction sites on the molecular sieve catalyst. The required water can be supplied by water of hydration in the molecular sieve catalysts or, alternatively, by the separate addition of water via the feed stream. When water of hydration is present, no added water is required since once the reaction is initiated, water produced during the course of the reaction (1 mole of water for each 2 moles of nitroaromatic compound produced) is sufficient to sustain it. If the molecular sieve catalyst is substantially anhydrous, it then becomes necessary to add water in an amount sufficient to provide the required reaction sites. The separate addition of water is usually preferred to ensure its presence in sufficient amounts. The amount of water present, however, is not narrowly critical. Thus, amounts ranging from nominal or trace amounts (eg. 0.1% by volume) up to 15% by volume of the feed stream are generally sufficient, with amounts ranging from 0.5% to 5% by volume being desirably used.

The vapor phase nitration of this invention is conveniently carried out in an apparatus of the type suitable for carrying out chemical reactions in the vapor phase. It can be conducted in a single reactor or in multiple reactors using either a fixed bed, moving bed, or a fluidized bed system to effect contacting of the reactants and the molecular sieve catalyst. The reaction is generally carried out by continuously passing a vaporous mixture of the aromatic compound and nitrogen dioxide over a bed of the molecular sieve catalyst while maintaining a temperature between about 80°C. and 190°C., and usually, 150°C. to 175°C.

The reactant aromatic compound can be preheated to form a vapor which is then admixed with gaseous nitrogen dioxide in a suitable reactor in predetermined relative proportions. Vaporous aromatic compounds can be conveniently swept into the reactor, at a constant rate by a water-containing stream of carrier gas and thence admixed with a continuous stream of nitrogen dioxide before contacting the heated catalyst bed. The reactants can be charged into the reactor at any suitable flow rate.

As previously indicated, the reactant materials are conveniently swept into the reactor by a stream of carrier gas. The carrier gas employed in the present process can be oxygen or an oxygen-containing gas, for example, air, or an inert gas such as nitrogen, and helium. It is advantageous, however, to employ oxygen or an oxygen-containing gas as the carrier gas due to the stoichiometry of the nitration reaction between the aromatic compound and the nitrogen dioxide.

In the initial nitration reaction between the aromatic compound and the nitrogen dioxide, it is believed that for each 2 moles of aromatic compound, 3 moles of nitrogen dioxide are required to produce 2 moles of nitroaromatic compound, 1 mole of nitric oxide, and 1 mole of water. In the absence of an oxygen source such as supplied by the oxygen-containing carrier gas, the nitric oxide is lost, thereby reducing the nitrogen dioxide selectivity to the nitroaromatic compound by at least 33% (one-third), as well as the material balance between reactants and recovered products. In the presence of oxygen (and the molecular sieve catalyst), however, the nitric oxide undergoes the known reoxidation to nitrogen dioxide (stoichiometrically requiring 1 mole of oxygen for each 2 moles of nitric oxide), which undergoes further reaction with additional aromatic compound. Overall, therefore, little, if any, nitrogen dioxide is lost by virtue of stoichiometrically produced nitric oxide.

The concentration of the aromatic compound in the feed mixture is not narrowly critical. All that is necessary is that the concentration be sufficient to permit the reaction to proceed at a reasonable rate. On the other hand, since the nitroaromatic compound produced will have a high vaporization temperature (in fact, higher than the operating temperature range, for example, nitrochlorobenzene isomers, b.p. 235—246°C.), the concentration should be such that the nitroaromatic compound produced will not condense in the reactor. In addition, since mixtures of aromatic compounds and air (the preferred aromatic compound carrier gas) are potentially flammable and explosive, it is necessary, from a practical viewpoint, to operate at concentrations outside the flammable and explosive limits of the aromatic compound being employed. Generally, concentrations between 1% and 15% by volume are desirably employed.

The relative proportions of reactants generally can range from 1 to 5 moles of nitrogen dioxide per mole of aromatic compound and, preferably, a ratio of 2:1 to 3:1 is used.

The present process is suited to either batch or continuous operations. Continuous operations can involve recirculation of the effluent stream unreacted aromatic compound and nitrogen dioxide following isolation of the nitroaromatic compound product. Additional reactants—aromatic compound and nitrogen dioxide—can then be charged to the reactor along with the recirculated stream to continue the process in a subsequent and continuous reaction. It will be noted that the substantial absence of side reactions such as, for example, the thermal decomposition of nitrogen dioxide and undesired by-product formation advantageously facilitates such continuous operations in that extensive purification of the effluent stream is not required and, as previously noted, the cost and complexity of reoxidation of the nitric oxide to nitrogen oxide to nitrogen dioxide is substantially reduced or eliminated altogether.

The nitroaromatic compounds produced during the course of the vapor phase reaction can be collected in a suitable chilled container, and purified by any appropriate method and means known to the art such as, for example, distillation and crystallization. Fractional crystallization in accordance with conventional procedures is especially convenient for the separation of ortho- and para- isomers when a monosubstituted aromatic compound having an ortho-para orientation substituent, such as chlorobenzene, is employed as the reactant or starting material.

The recovered unreacted reactants, due to the substantial absence of side-reactions to produce undesired by-products, are easily recycled to the reactor for further processing.

The following specific examples illustrating the best presently-known methods of practicing this invention are described in detail in order to facilitate a clear understanding of the invention.

Examples 1—21

A stainless steel tube 40.64 cm (16 inches) in length and 2.54 cm (1 inch) outside diameter, packed with a 35.56 cm (14 inch) bed of molecular sieve catalyst was employed as the reactor. The catalyst, unless specified otherwise, was pretreated with nitrogen dioxide at operating conditions (in the absence of the aromatic compounds) to the saturation point, usually from about 5 minutes to about 1 hour.

A stream of aromatic compound was preheated and charged to the reactor tube in a humidified or water-containing stream of air. The nitrating agent, nitrogen dioxide unless otherwise specified, in a nitrogen carrier stream was mixed with the aromatic compound/air stream shortly before contact with the heated catalyst.

The products were collected in a series of three chilled containers, the first of which was chilled in an ice water bath and the second and third of which were chilled in dry ice baths. Analyses were performed by gas chromatography on a Varian Associates Model 3700 instrument using a 1.83 meter (6 ft.)×0.32 cm (0.125 inch) outside diameter SP-1000 on 0.5% phosphoric acid treated Chrom. G. Column programmed from 90°C. to 210°C. at a program rate of 10°C./minute.

The parameters and the results are tabulated in Table 1.

TABLE 1

| Example | Catalyst[1] | | Aromatic Compound, R—$C_6H_5$ | | | | | | | Carrier gas |
| | Type | $SiO_2/Al_2O_3$ molar ratio | Pretreat. Time min. | R | Flow Rate ml/min. | g, moles | Conc. vol.% | Temp. °C. | Flow rate ml/min. |
|---|---|---|---|---|---|---|---|---|---|
| 1 | Zeolon 900 H | 10/1 | 30 | Cl | 18.16 | 30.1, 0.27 | 2.5 | 75 | 500.0 |
| 2 | " | " | 15 | " | 18.27 | 27.8, 0.25 | 2.5 | " | " |
| 3 | " | " | 15 | " | 16.13 | 24.3, 0.22 | 2.3 | " | " |
| 4 | " | " | 16 | " | 20.11 | 30.3, 0.27 | 2.7 | " | " |
| 5 | " | " | 24 | " | 21.44 | 32.3, 0.29 | 3.1 | " | " |
| 6 | " | " | 15 | " | 37.99 | 68.1, 0.61 | 3.6 | " | 800.0 |
| 7 | " | " | 17 | " | 26.64 | 40.8, 0.36 | 3.8 | 81 | 500.0 |
| 8 | " | " | 20 | " | 18.98 | 28.6, 0.25 | 2.6 | 75 | " |
| 9 | " | " | 20 | " | 19.31 | 29.1, 0.26 | 2.6 | " | " |
| 10 | " | " | 25 | " | 49.41 | 67.0, 0.60 | 4.9 | 81 | 770.0 |
| 11 | " | " | 25 | " | 79.50 | 107.8, 0.96 | 7.2 | " | 850.0 |

TABLE 1 (Cont'd)

| | Catalyst[1] | | | Aromatic Compound, R—C₆H₅ | | | | | Carrier gas |
|---|---|---|---|---|---|---|---|---|---|
| Example | Type | SiO₂/Al₂O₃ molar ratio | Pretreat. Time min. | R | Flow Rate ml/min. | g, moles | Conc. vol.% | Temp. °C. | Flow rate ml/min. |
| 12[6] | ʺ | ʺ | — | ʺ | 33.16 | 51.6, 0.46 | 4.5 | 85 | 500.0 |
| 13 | ʺ | ʺ | 37 | ʺ | 19.91 | 32.0, 0.28 | 2.7 | ʺ | ʺ |
| 14[6] | ʺ | ʺ | 15 | ʺ | 20.57 | 31.0, 0.28 | 2.8 | 75 | ʺ |
| 15 | Zeolon 200 H | 10/1 | 10 | ʺ | 17.46 | 26.3, 0.23 | 2.4 | ʺ | ʺ |
| 16[6] | Silica-alumina | 7.25/1 | — | ʺ | 22.56 | 27.2, 0.24 | 3.2 | 80 | ʺ |
| 17 | ʺ | ʺ | 7 | ʺ | 24.62 | 44.5, 0.40 | 3.4 | ʺ | ʺ |
| 18 | ʺ | ʺ | 10 | ʺ | 31.58 | 57.1, 0.51 | 4.4 | ʺ | ʺ |
| 19 | ʺ | ʺ | 7 | ʺ | 31.73 | 47.8, 0.42 | 4.3 | ʺ | ʺ |
| 20 | ʺ | ʺ | 5 | H | 77.59 | 41.9, 0.54 | 8.6 | 30 | ʺ |
| 21 | ʺ | ʺ | 2 | ʺ | 82.77 | 40.4, 0.52 | 9.2 | ʺ | ʺ |

### TABLE 1 (Cont'd)

| Example | Nitrating Agent[2] | | | | Carrier Gas[5] | Nitrating agent/aromatic compound |
| | Flow rate ml/min. | g, moles | Conc. vol. % | Temp. °C. | Flow rate ml/min. | molar ratio |
|---|---|---|---|---|---|---|
| 1 | 65.96 | 44.7, 0.97 | 9.2 | 15 | 31.2 | 3.63 |
| 2 | 65.85 | 41.0, 0.89 | 9.1 | " | " | 3.60 |
| 3 | 49.16 | 30.3, 0.66 | 7.0 | " | " | 3.00 |
| 4 | 75.66 | 46.6, 1.01 | 10.2 | " | " | 3.74 |
| 5 | 49.75 | 30.7, 0.67 | 7.1 | " | 21.2 | 2.31 |
| 6 | 71.42 | 46.2, 1.00 | 6.8 | " | 31.2 | 1.64 |
| 7 | 43.27 | 27.1, 0.59 | 6.2 | " | 21.2 | 1.64 |
| 8 | 67.40 | 41.5, 0.90 | 9.2 | " | 31.2 | 3.60 |
| 9 | 79.89 | 49.2, 1.07 | 10.7 | " | " | 4.12 |
| 10 | 51.21 | 28.4, 0.62 | 5.1 | " | 23.7 | 1.03 |
| 11 | 47.21 | 26.2, 0.57 | 4.3 | " | 21.2 | 0.59 |

TABLE 1 (Cont'd)

| Example | Nitrating Agent[2] | | | | Carrier Gas[5] | Nitrating agent/aromatic compound |
| | Flow rate ml/min. | g, moles | Conc. vol. % | Temp. °C. | Flow rate ml/min. | molar ratio |
|---|---|---|---|---|---|---|
| 12[6] | 63.60 | 40.5, 0.88 | 8.6 | 15 | 31.0 | 1.91 |
| 13 | 72.70 | 47.8, 1.00 | 10.6 | „ | 21.2 | 3.70 |
| 14[6] | 69.56 | 42.9, 0.93 | 9.5 | „ | 31.2 | 3.30 |
| 15 | 78.08 | 48.1, 1.05 | 10.6 | „ | „ | 4.57 |
| 16[6] | 54.88 | 27.0, 0.57 | 7.7 | „ | 30.0 | 2.38 |
| 17 | 46.41 | 34.3, 0.75 | 6.5 | „ | 26.0 | 1.88 |
| 18 | 37.89 | 28.0, 0.61 | 5.3 | „ | 21.0 | 1.20 |
| 19 | 76.14 | 46.9, 1.02 | 10.2 | „ | 30.0 | 2.43 |
| 20 | 145.14 | 46.2, 1.00 | 16.1 | „ | 69.0 | 1.85 |
| 21 | 135.15 | 38.8, 0.84 | 15.1 | „ | „ | 1.62 |

TABLE 1 (Cont'd)

| Example | Water | | | | Carrier Gas[4] | Reaction Conditions | | |
| | Flow rate ml/min. | g, moles | Conc. vol.% | Temp. °C. | Flow rate ml/min. | Temp. °C. | Time hours | Conversion[3]% |
|---|---|---|---|---|---|---|---|---|
| 1 | 4.53 | 1.2, 0.07 | 0.6 | 81 | 99.8 | 175 | 5.5 | 43.3 |
| 2 | 11.74 | 2.9, 0.16 | 1.6 | " | " | " | 5.05 | 85.0 |
| 3 | 9.09 | 2.2, 0.12 | 1.2 | " | " | " | 5.0 | 75.7 |
| 4 | 11.81 | 2.8, 0.16 | 1.6 | " | " | " | 5.0 | 80.5 |
| 5 | 10.75 | 2.5, 0.14 | 1.5 | " | " | " | 5.0 | 62.0 |
| 6 | 14.35 | 3.6, 0.20 | 1.4 | " | " | " | 5.25 | 43.9 |
| 7 | 11.99 | 2.9, 0.16 | 1.7 | " | " | " | 5.08 | 47.7 |
| 8 | 14.78 | 3.6, 0.20 | 2.0 | " | " | " | 5.0 | 59.6 |
| 9 | 13.61 | 3.3, 0.18 | 1.8 | " | " | " | 5.0 | 49.4 |
| 10 | 10.73 | 2.3, 0.13 | 1.1 | " | " | " | 4.5 | 20.8 |
| 11 | 12.66 | 2.8, 0.16 | 1.1 | " | " | " | 4.5 | 12.8 |

TABLE 1 (Cont'd)

| Example | Water | | | | Carrier Gas[4] | Reaction Conditions | | |
|---|---|---|---|---|---|---|---|---|
| | Flow rate ml/min. | g, moles | Conc. vol.% | Temp. °C. | Flow rate ml/min. | Temp. °C. | Time hours | Conversion[3]% |
| 12[6] | 14.97 | 3.7, 0.21 | 2.0 | 85 | 98.0 | " | 5.2 | 54.3 |
| 13 | 11.15 | 2.9, 0.16 | 1.5 | 81 | 99.8 | 100 | 5.33 | 28.9 |
| 14[6] | 11.46 | 2.8, 0.16 | 1.6 | " | " | 200 | 5.0 | 83.9 |
| 15 | 9.96 | 2.4, 0.13 | 1.4 | " | " | 175 | 5.0 | 66.3 |
| 16[6] | 7.99 | 1.5, 0.083 | 1.1 | 80 | 98 | " | 4.0 | 50.8 |
| 17 | 24.01 | 7.0, 0.39 | 3.3 | 90 | " | " | 6.0 | 75.7 |
| 18 | 23.75 | 6.9, 0.38 | 3.3 | " | " | " | 6.0 | 49.3 |
| 19 | 9.71 | 2.3, 0.13 | 1.3 | 80 | " | 150 | 5.0 | 33.7 |
| 20 | 12.04 | 1.5, 0.083 | 1.3 | " | " | " | 2.6 | 92.4 |
| 21 | 11.38 | 1.3, 0.072 | 1.3 | " | " | 130 | 2.3 | 70.5 |

### TABLE 1 (Cont'd)

Products, %

| Example | R=H | R—$C_6H_4$—$NO_2$ R=Cl | | | Unidentified by-products | Material Balance g | | |
|---|---|---|---|---|---|---|---|---|
| | | ortho | meta | para | | In | Out | % |
| 1 | — | 9.9 | 0.3 | 33.0 | <0.1 | 76.0 | 76.4 | 100.5 |
| 2 | — | 12.5 | 1.3 | 71.7 | <0.1 | 7.6 | 65.0 | 90.8 |
| 3 | — | 13.8 | 1.3 | 60.5 | <0.1 | 56.8 | 53.7 | 94.5 |
| 4 | — | 15.5 | 1.3 | 63.2 | 0.5 | 79.8 | 79.1 | 99.1 |
| 5 | — | 12.3 | 1.1 | 48.5 | 0.1 | 65.5 | 64.2 | 98.0 |
| 6 | — | 9.9 | 0.6 | 33.3 | <0.1 | 109.9 | 105.2 | 95.7 |
| 7 | — | 10.6 | 0.8 | 36.2 | <0.1 | 70.8 | 70.5 | 99.6 |
| 8 | — | 13.4 | 0.2 | 45.9 | 0.2 | 74.4 | 73.7 | 99.0 |
| 9 | — | 12.0 | 0.1 | 37.3 | — | 81.6 | 79.2 | 97.1 |
| 10 | — | 5.0 | 0.5 | 15.3 | <0.1 | 97.7 | 96.8 | 99.1 |
| 11 | — | 3.0 | 0.2 | 9.6 | <0.1 | 136.7 | 134.4 | 98.3 |

## TABLE 1 (Cont'd)

### Products, %

| Example | R=H | R—C$_6$H$_4$—NO$_2$ R=Cl | | | Unidentified by-products | Material Balance g | | |
|---|---|---|---|---|---|---|---|---|
| | | ortho | meta | para | | In | Out | % |
| 12[6] | — | 10.4 | 1.1 | 42.3 | 0.6 | 95.8 | 80.3 | 83.8 |
| 13 | — | 7.6 | 0.2 | 21.2 | — | 82.7 | 81.9 | 99.0 |
| 14[6] | — | 11.6 | 2.8 | 66.0 | 3.5 | 76.6 | 71.1 | 92.8 |
| 15 | — | 17.2 | 0.7 | 48.3 | <0.1 | 76.8 | 72.2 | 94.0 |
| 16[6] | — | 14.4 | 0.6 | 34.3 | — | 55.7 | 49.7 | 89.2 |
| 17 | — | 21.2 | 1.2 | 52.9 | 0.4 | 85.8 | 78.3 | 91.3 |
| 18 | — | 13.5 | 0.8 | 34.8 | 0.2 | 92.0 | 86.9 | 94.5 |
| 19 | — | 9.3 | 0.0 | 23.9 | 0.5 | 97.0 | 90.7 | 93.5 |
| 20 | 92.4 | — | — | — | — | 89.6 | 88.6 | 98.9 |
| 21 | 70.5 | — | — | — | — | 80.5 | 72.6 | 90.2 |

[1]Pretreated for the indicated period of time with nitrogen dioxide at operating conditions (in the absence of the aromatic compound) unless specified otherwise.
[2]Nitrogen dioxide (M.W., 46) unless specified otherwise.
[3]Based on the aromatic compound.
[4]Air
[5]Nitrogen
[6]Comparative example.
[7]A silica/alumina composition (SiO$_2$/Al$_2$O$_3$ molar ratio—12/1) available from Strem Chemicals of Newburyport, Massachusetts, U.S.A.

Thus, it is apparent that there has been provided, in accordance with the present invention, a process that fully satisfies the objects and advantages set forth hereinabove.

**Claims**

1. A process for the vapor phase nitration of aromatic compounds susceptible of existing in the vapor phase at temperatures less than 190°C, which process comprises contacting the aromatic compound with a gaseous oxide of nitrogen higher than nitric oxide as nitrating agent selected from nitrogen dioxide, dinitrogen trioxide, and dinitrogen tetroxide in the vapor phase in the presence of water and a molecular sieve catalyst, characterised in that the process is carried out at a temperature between 80°C and 190°C and the molecular sieve catalyst is conditioned by pretreatment with the nitrating agent.

2. The process of Claim 1 wherein the nitrating agent is nitrogen dioxide.

3. The process of Claim 1 wherein the nitrating agent is admixed with a carrier gas prior to reaction with the aromatic compound.

4. The process of Claim 3 wherein the carrier gas is nitrogen.

5. The process of Claim 1 wherein the catalyst pretreatment with the nitrating agent is carried out for 1 minute to 1 hour.

6. The process of Claim 1 wherein the vapor phase reaction is carried out at temperatures ranging from 150°C to 175°C.

7. The process of Claim 1 wherein 1 to 5 moles of nitrating agent are used per mole of aromatic compound.

8. The process of Claim 1 wherein the aromatic compound is an aromatic hydrocarbon.

9. The process of Claim 9 wherein the aromatic hydrocarbon is benzene or toluene.

10. The process of Claim 1 wherein the aromatic compound is a haloaromatic compound.

11. The process of Claim 10 wherein the haloaromatic compound is selected from chlorobenzene, bromobenzene, and iodobenzene.

12. The process of Claim 1 wherein the concentration of the aromatic compound in the feed mixture is between 1% and 15% by volume.

13. The process of Claim 1 wherein the aromatic compound is admixed with a carrier gas prior to reaction with the nitrating agent.

14. The process of Claim 13 wherein the carrier gas is an oxygen-containing gas.

15. The process of Claim 14 wherein the oxygen-containing gas is air.

16. The process of Claim 1 wherein water vapor is admixed with the feed mixture prior to reaction between the aromatic compound and the nitrating agent.

17. The process of Claim 16 wherein the water vapor is present in the feed mixture in a concentration ranging from 0.1% up to 15% by volume.

18. The process of Claim 1 wherein the aromatic compound is chlorobenzene, the nitrating agent is nitrogen dioxide, the molar ratio of nitrogen dioxide to chlorobenzene is from 1:1 to 5:1, and the temperature ranges from 150°C. to 175°C.

19. The process of Claim 1 wherein the aromatic compound is benzene, the nitrating agent is nitrogen dioxide, the molar ratio of nitrogen dioxide to benzene is from 1:1 to 5:1, and the temperature ranges from 150°C to 175°C.

**Patentansprüche**

1. Verfahren zur Dampfphasen-Nitrierung von aromatischen Verbindungen, die in der Dampfphase bei Temperaturen von weniger als 190°C existieren können, wobei die aromatische Verbindung mit einem gasförmigen Oxid des Stickstoffs, welches höher als Stickstoff(II)-oxid ist, als Nitrierungsmittel, ausgewählt unter Stickstoffdioxid, Distickstofftrioxid und Distickstofftetroxid in der Dampfphase in Gegenwart von Wasser und eines Molekularsieb-Katalysators in Berührung gebracht wird, dadurch gekennzeichnet, daß das Verfahren bei einer Temperatur zwischen 80°C und 190°C durchgeführt wird und der Molekularsieb-Katalysator durch Vorbehandlung mit dem Nitrierungsmittel konditioniert ist.

2. Verfahren nach Anspruch 1, worin das Nitrierungsmittel Stickstoffdioxid ist.

3. Verfahren nach Anspruch 1, worin das Nitrierungsmittel mit einem Trägergas vor der Reaktion mit der aromatischen Verbindung vermischt wird.

4. Verfahren nach Anspruch 3, worin das Trägergas Stickstoff ist.

5. Verfahren nach Anspruch 1, worin die Katalysator-Vorbehandlung mit dem Nitrierungsmittel für 1 Minute bis zu 1 Stunde durchgeführt wird.

6. Verfahren nach Anspruch 1, worin die Dampfphasen-Reaktion bei einer Temperatur im Bereich von 150°C bis 175°C durchgeführt wird.

7. Verfahren nach Anspruch 1, worin 1 bis 5 Mol Nitrierungsmittel pro Mol aromatischer Verbindung verwendet werden.

8. Verfahren nach Anspruch 1, worin die aromatische Verbindung ein aromatischer Kohlenwasserstoff ist.

9. Verfahren nach Anspruch 9, worin der aromatische Kohlenwasserstoff Benzol oder Toluol ist.

10. Verfahren nach Anspruch 1, worin die aromatische Verbindung eine halogenaromatische Verbindung ist.

11. Verfahren nach Anspruch 10, worin die halogenaromatische Verbindung unter Chlorbenzol, Brombenzol und Jodbenzol ausgewählt ist.

12. Verfahren nach Anspruch 1, worin die Konzentration der aromatischen Verbindung in dem Speisegemisch zwischen 1 Vol.-% und 15 Vol.-% liegt.

13. Verfahren nach Anspruch 1, worin die aromatische Verbindung mit einem Trägergas vor der Reaktion mit dem Nitrierungsmittel vermischt wird.

14. Verfahren nach Anspruch 13, worin das Trägergas ein Sauerstoff enthaltendes Gas ist.

15. Verfahren nach Anspruch 14, worin das Sauerstoff enthaltende Gas Luft ist.

16. Verfahren nach Anspruch 1, worin Wasserdampf mit dem Speisegemisch vor der Reaktion zwischen der aromatischen Verbindung und dem Nitrierungsmittel vermischt wird.

17. Verfahren nach Anspruch 16, worin der Wasserdampf in dem Speisegemisch in einer Konzentration im Bereich von 0,1 Vol.-% bis 15 Vol.-% vorhanden ist.

18. Verfahren nach Anspruch 1, worin die aromatische Verbindung Chlorbenzol ist, das Nitrierungsmittel Stickstoffdioxid ist, das Molverhältnis von Stickstoffdioxid zu Chlorbenzol von 1:1 bis 5:1 beträgt, und die Temperatur im Bereich von 150°C bis 175°C liegt.

19. Verfahren nach Anspruch 1, worin die aromatische Verbindung Benzol ist, das Nitrierungsmittel Stickstoffdioxid ist, das Molverhältnis von Stickstoffdioxid zu Benzol von 1:1 bis 5:1 beträgt, und die Temperatur im Bereich von 150°C bis 175°C liegt.

## Revendications

1. Procédé pour la nitration en phase vapeur de composés aromatiques susceptibles d'exister en phase vapeur à des températures inférieures à 190°C, qui consiste à mettre en contact le composé aromatique avec un oxyde gazeux d'azote supérieur au bioxyde d'azote (ou oxyde nitrique), en tant qu'agent de nitration choisi parmi le peroxyde d'azote $NO_2$, l'anhydride azoteux $N_2O_3$ et la peroxyde d'azote (forme dimère) $N_2O_4$ en phase vapeur en présence d'eau et d'un catalyseur formé de tamis moléculaire, caractérisé en ce que le procédé est réalisé à une température entre 80°C et 190°C et en ce que le catalyseur formé de tamis moléculaire est conditionné par pré-traitement avec l'agent de nitration.

2. Procédé selon la revendication 1, dans lequel l'agent de nitration est le peroxyde d'azote $NO_2$.

3. Procédé selon la revendication 1, dans lequel l'agent de nitration est mélangé avec un gaz porteur avant la réaction avec le composé aromatique.

4. Procédé selon la revendication 3, dans lequel le gaz porteur est l'azote.

5. Procédé selon la revendication 1, dans lequel le pré-traitement du catalyseur avec l'agent de nitration est réalisé pendant 1 minute à 1 heure.

6. Procédé selon la revendication 1, dans lequel la réaction en phase vapeur est réalisée à des températures allant de 150°C à 175°C.

7. Procédé selon la revendication 1, dans lequel 1 à 5 moles d'agent de nitration sont utilisées par mole de composé aromatique.

8. Procédé selon la revendication 1, dans lequel le composé aromatique est un hydrocarbure aromatique.

9. Procédé selon la revendication 8, dans lequel l'hydrocarbure aromatique est le benzène ou le toluène.

10. Procédé selon la revendication 1, dans lequel le composé aromatique est un composé halo-aromatique.

11. Procédé selon la revendication 10, dans lequel le composé haloaromatique est choisi parmi le chlorobenzène, le bromobenzène et l'iodobenzène.

12. Procédé selon la revendication 1, dans lequel la concentration du composé aromatique dans le mélange d'alimentation est comprise entre 1% et 15% en volume.

13. Procédé selon la revendication 1, dans lequel le composé aromatique est mélangé avec un gaz porteur avant la réaction avec l'agent de nitration.

14. Procédé selon la revendication 13, dans lequel le gaz porteur est un gaz contenant de l'oxygène.

15. Procédé selon la revendication 14, dans lequel le gaz contenant de l'oxygène est l'air.

16. Procédé selon la revendication 1, dans lequel la vapeur d'eau est mélangée avec le mélange d'alimentation avant la réaction entre le composé aromatique et l'agent de nitration.

17. Procédé selon la revendication 16, dans lequel la vapeur d'eau est présente dans le mélange d'alimentation à une concentration allant de 0,1% jusqu'à 15% en volume.

18. Procédé selon la revendication 1, dans lequel le composé aromatique est le chlorobenzène, l'agent de nitration est le peroxyde d'azote $NO_2$, le rapport molaire peroxyde d'azote $NO_2$/chlorobenzène est de 1:1 à 5:1, et la température va de 150°C à 175°C.

19. Procédé selon la revendication 1, dans lequel le composé aromatique est le benzène, l'agent de nitration est le peroxyde d'azote $NO_2$, le rapport molaire peroxyde d'azote $NO_2$/benzène est de 1:1 à 5:1, et la température va de 150°C à 175°C.